# EUROPEAN PATENT APPLICATION

(11) **EP 4 603 028 A1**
(43) Date of publication of application: **20.08.2025**
(21) Application number: 23885293.3
(22) Date of filing: 07.06.2023
(51) Int. Cl.: A61B 10/00, G01N 21/64

(54) **LYMPHATIC SYSTEM INSPECTION DEVICE**

(30) Priority: 04.11.2022 JP 2022177496
(71) Applicant: Hamamatsu Photonics K.K., Hamamatsu-shi, Shizuoka 435-8558 (JP)
(72) Inventor: SHIKAYAMA Takahiro, Hamamatsu-shi, Shizuoka 435-8558 (JP); MURAKOSHI Takahiro, Hamamatsu-shi, Shizuoka 435-8558 (JP); SATO Takayuki, Hamamatsu-shi, Shizuoka 435-8558 (JP); MIZUNO Toshihiko, Hamamatsu-shi, Shizuoka 435-8558 (JP); SHINAOKA Akira, Okayama-shi, Okayama 700-8530 (JP)
(74) Representative: Hoffmann Eitle
(86) International application number: PCT/JP2023/021080
(87) International publication number: WO 2024/095519

(57) **Abstract**

A lymphatic system examination device includes a main body portion configured to position a lower body of a subject in an imaging region, an illumination unit configured to irradiate the lower body with excitation light; and an imaging unit configured to detect fluorescence emitted from the lower body. The illumination unit and the imaging unit are arranged on one side in a first horizontal direction. The illumination unit includes a first light emitting region and a second light emitting region arranged in a state of being separated from each other in a second horizontal direction. Each of the first light emitting region and the second light emitting region extends in a vertical direction in a state of facing the imaging region. The imaging unit faces the imaging region across a region between the first light emitting region and the second light emitting region.

## Description

### Technical Field

The present disclosure relates to a lymphatic system examination device.

### Background Art

The lymphatic system is a network that functions as a circulatory system of lymphatic fluid and is composed of lymph nodes, lymphatic vessels, and the like. As an examination method for diagnosing a disease of the lymphatic system (for example, lymphedema), a method is known in which a predetermined part of a subject into which a fluorescent dye such as indocyanine green is injected into the lymphatic system is irradiated with excitation light, and fluorescence emitted from the predetermined part in response to the irradiation of the excitation light is detected to acquire a fluorescent image of the predetermined part (see, for example, Patent Literature 1).

### Citation List

### Patent Literature

Patent Literature 1: Japanese Unexamined Patent Publication No. 2019-118803

### Summary of Invention

### Technical Problem

The device used for the examination as described above has a problem that the irradiation range of the excitation light and the detection range of the fluorescence are limited to narrow ranges, and it is difficult to efficiently examine the lymphatic system in the lower body of the subject.

An object of the present disclosure is to provide a lymphatic system examination device suitable for examination of a lymphatic system in the lower body of a subject.

### Solution to Problem

A lymphatic system examination device according to an aspect of the present disclosure is [1] "a lymphatic system examination device including a main body portion including a placement portion on which a subject in which a fluorescent dye is injected into a lymphatic system stands, and configured to position a lower body of the subject in an imaging region on the placement portion; an illumination unit configured to irradiate the lower body with excitation light; and an imaging unit configured to detect fluorescence emitted from the lower body in response to irradiation of the excitation light, in which the illumination unit and the imaging unit are arranged on one side of the imaging region in a first horizontal direction, the illumination unit includes a first light emitting region and a second light emitting region arranged in a state of being separated from each other in a second horizontal direction perpendicular to the first horizontal direction, each of the first light emitting region and the second light emitting region extends in a vertical direction in a state of facing the imaging region, and the imaging unit faces the imaging region across a region between the first light emitting region and the second light emitting region".

In the lymphatic system examination device according to [1], each of the first light emitting region and the second light emitting region in the illumination unit extends in the vertical direction in a state of facing the imaging region, and the imaging unit faces the imaging region across a region between the first light emitting region and the second light emitting region. As a result, the lower body of the subject positioned in the imaging region can be uniformly irradiated with the excitation light, and the fluorescence emitted from the lower body in response to the uniform irradiation of the excitation light can be detected. Therefore, according to the lymphatic system examination device according to [1], it is possible to acquire a fluorescent image of the lower body appropriate for examination of the lymphatic system in the lower body of the subject.

The lymphatic system examination device according to an aspect of the present disclosure may be [2] "the lymphatic system examination device according to [1], in which the imaging unit is positioned in a center of the imaging region when viewed from the first horizontal direction". According to the lymphatic system examination device according to [2], it is possible to suppress occurrence of distortion in the acquired fluorescent image.

The lymphatic system examination device according to an aspect of the present disclosure may be [3] "the lymphatic system examination device according to [1] or [2], in which the illumination unit is positioned between the imaging region and the imaging unit in the first horizontal direction". According to the lymphatic system examination device according to [3], it is possible to uniformly irradiate the lower body of the subject positioned in the imaging region with excitation light.

The lymphatic system examination device according to an aspect of the present disclosure may be [4] "the lymphatic system examination device according to any one of [1] to [3], in which the first light emitting region includes a plurality of first light emitting portions, the second light emitting region includes a plurality of second light emitting portions, and the illumination unit has a function of adjusting a light emission intensity of each of the plurality of first light emitting portions and a light emission intensity of the plurality of second light emitting portions". According to the lymphatic system examination device according to [4], it is possible to uniformly irradiate the lower body with the excitation light in response to a shape and the like of the lower body of the subject positioned in the imaging region.

The lymphatic system examination device according to an aspect of the present disclosure may be [5] "the lymphatic system examination device according to any one of [1] to [4], in which the first light emitting region and the second light emitting region are arranged so as to be away from each other in the second horizontal direction as approaching the imaging region in the first horizontal direction". According to the lymphatic system examination device according to [5], it is possible to uniformly irradiate the lower body of the subject positioned in the imaging region with excitation light.

The lymphatic system examination device according to an aspect of the present disclosure is [6] "a lymphatic system examination device including a main body portion including a placement portion on which a subject in which a fluorescent dye is injected into a lymphatic system stands, and configured to position a lower body of the subject in an imaging region on the placement portion; an illumination unit configured to irradiate the lower body with excitation light; an imaging unit configured to acquire an excitation light image of the lower body by the excitation light and a fluorescence image of the lower body by fluorescence emitted from the lower body in response to irradiation with the excitation light; and a control unit configured to generate excitation light correction data based on the excitation light image of the lower body and correct the fluorescence image of the lower body based on the excitation light correction data.

In the lymphatic system examination device according to [6], the excitation light image of the lower body and the fluorescence image of the lower body are acquired, the excitation light correction data is generated based on the excitation light image of the lower body, and the fluorescence image of the lower body is corrected based on the excitation light correction data. As a result, it is possible to suppress the occurrence of unevenness in the fluorescent image of the lower body due to the non-uniformity of the irradiation intensity of the excitation light in the lower body. Therefore, according to the lymphatic system examination device according to [6], it is possible to acquire a fluorescent image of the lower body appropriate for examination of the lymphatic system in the lower body of the subject.

The lymphatic system examination device according to an aspect of the present disclosure may be [7] "the lymphatic system examination device according to [6], in which the imaging unit acquires an excitation light image of the plate by the excitation light and a fluorescence image of the plate by fluorescence emitted from the plate in response to irradiation of the excitation light in a state in which a plate for luminance calibration is positioned in the imaging region, the imaging unit uses a filter that selectively transmits the fluorescence when the fluorescence image of the plate is acquired, and the control unit generates filter correction data based on the excitation light image of the plate and the fluorescence image of the plate and corrects the fluorescence image of the lower body based on the excitation light correction data and the filter correction data". According to the lymphatic system examination device according to [7], it is possible to suppress the occurrence of unevenness in the fluorescent image of the lower body due to the angular dependence of the transmission wavelength in the filter.

The lymphatic system examination device according to an aspect of the present disclosure may be [8] "the lymphatic system examination device according to [6] or [7], in which the control unit corrects a contour of the fluorescence image of the lower body in the fluorescent image of the lower body". According to the lymphatic system examination device according to [8], it is possible to suppress the emphasis of the contour of the fluorescent image of the lower body by the correction based on the excitation light correction data.

The lymphatic system examination device according to an aspect of the present disclosure may be [9] "the lymphatic system examination device according to any one of [6] to [8], further including a display unit configured to display the fluorescence image of the lower body corrected by the control unit, in which the control unit generates a plurality of images arranged in time series as the corrected fluorescence image of the lower body and controls the display unit so that the plurality of images is continuously displayed". According to the lymphatic system examination device according to [9], the fluorescent image of the lower body can be continuously grasped in time series.

A lymphatic system examination method according to an aspect of the present disclosure is [10] "a lymphatic system examination method performed in a lymphatic system examination device, in which the lymphatic system examination device includes a main body portion including a placement portion on which a subject in which a fluorescent dye is injected into a lymphatic system stands, and configured to position a lower body of the subject in an imaging region on the placement portion; and an illumination unit configured to irradiate the lower body with excitation light; an imaging unit configured to acquire an excitation light image of the lower body by the excitation light and a fluorescence image of the lower body by fluorescence emitted from the lower body in response to irradiation with the excitation light, and the lymphatic system examination method includes a step of acquiring the excitation light image of the lower body and the fluorescence image of the lower body, and a step of generating excitation light correction data based on the excitation light image of the lower body and correcting the fluorescence image of the lower body based on the excitation light correction data.

In the lymphatic system examination method according to [10], the excitation light image of the lower body and the fluorescence image of the lower body are acquired, the excitation light correction data is generated based on the excitation light image of the lower body, and the fluorescence image of the lower body is corrected based on the excitation light correction data. As a result, it is possible to suppress the occurrence of unevenness in the fluorescent image of the lower body due to the non-uniformity of the irradiation intensity of the excitation light in the lower body. Therefore, according to the lymphatic system examination method according to [10], it is possible to acquire a fluorescent image of the lower body appropriate for examination of the lymphatic system in the lower body of the subject.

### Advantageous Effects of Invention

According to the present disclosure, it is possible to provide a lymphatic system examination device suitable for examination of a lymphatic system in the lower body of a subject.

### Brief Description of Drawings

FIG. 1 is a perspective view of a lymphatic system examination device according to an embodiment.
FIG. 2 is a perspective view of a device main body illustrated in FIG. 1.
FIG. 3 is a cross-sectional view of the device main body taken along line III-III illustrated in FIG. 2.
FIG. 4 is a cross-sectional view of the device main body taken along line IV-IV illustrated in FIG. 3.
FIG. 5 is a cross-sectional view of the device main body taken along line V-V illustrated in FIG. 3.
FIG. 6 is a cross-sectional view of a restricting portion illustrated in FIG. 2.
FIG. 7 is a cross-sectional view of the device main body in which a second plate for luminance calibration is disposed.
FIG. 8 is a diagram illustrating a visible-light image of an object.
FIG. 9 is a diagram illustrating a display unit on which a plurality of types of fluorescent images of the object illustrated in FIG. 8 are displayed.
FIG. 10 is a plan view of a device main body of a modification.
FIG. 11 is a configuration diagram of an imaging unit according to an embodiment.
FIG. 12 is a flowchart illustrating processing of generating filter correction data.
FIG. 13 is a diagram illustrating the processing of generating the filter correction data.
FIG. 14 is a diagram illustrating a specific example of the processing of generating the filter correction data.
FIG. 15 is a flowchart illustrating processing of generating a fluorescence image of the subject.
FIG. 16 is a diagram illustrating processing of generating the fluorescence image of the subject.
FIG. 17 is a diagram illustrating a specific example of the processing of generating the fluorescence image of the subject.
FIG. 18 is a diagram illustrating the effect of processing of generating the fluorescence image of the subject.
FIG. 19 is a configuration diagram of an imaging unit according to the modification.

### Description of Embodiments

Hereinafter, embodiments of the present disclosure are described in detail with reference to the accompanying drawings. Note that, in each drawing, the same or corresponding parts are denoted by the same reference signs, and redundant description is omitted.

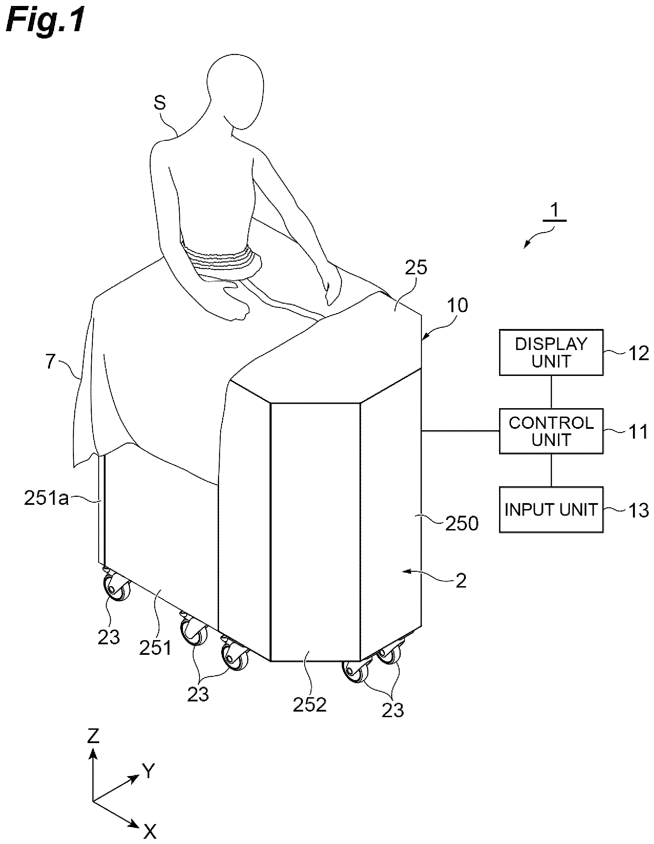

A lymphatic system examination device 1 illustrated in FIG. 1 is a device that examines the lymphatic system in the lower body of a subject S in order to diagnose a disease (for example, lymphedema) of the lymphatic system. More specifically, the lymphatic system examination device 1 is a device that irradiates the lower body of the subject S in which a fluorescent dye such as indocyanine green is injected into the lymphatic system with excitation light and detects fluorescence emitted from the lower body in response to the irradiation of the excitation light, thereby acquiring a fluorescent image of the lower body. Hereinafter, the vertical direction is referred to as a Z direction, the first horizontal direction is referred to as an X direction, and the second horizontal direction perpendicular to the first horizontal direction is referred to as a Y direction.

As illustrated in FIG. 1, the lymphatic system examination device 1 includes a device main body 10, a control unit 11, a display unit 12, and an input unit 13. The control unit 11 is composed of a processing unit and a storage unit. The processing unit of the control unit 11 is a computer device that is composed of a processor, a memory, a storage, a communication device, and the like and processes various items of data by executing software (program). The storage unit of the control unit 11 is a hard disk or the like and stores various items of data. The display unit 12 is a display or the like and displays various items of data to the operator. The input unit 13 is a mouse, a keyboard, or the like and receives inputs of various items of data from an operator.

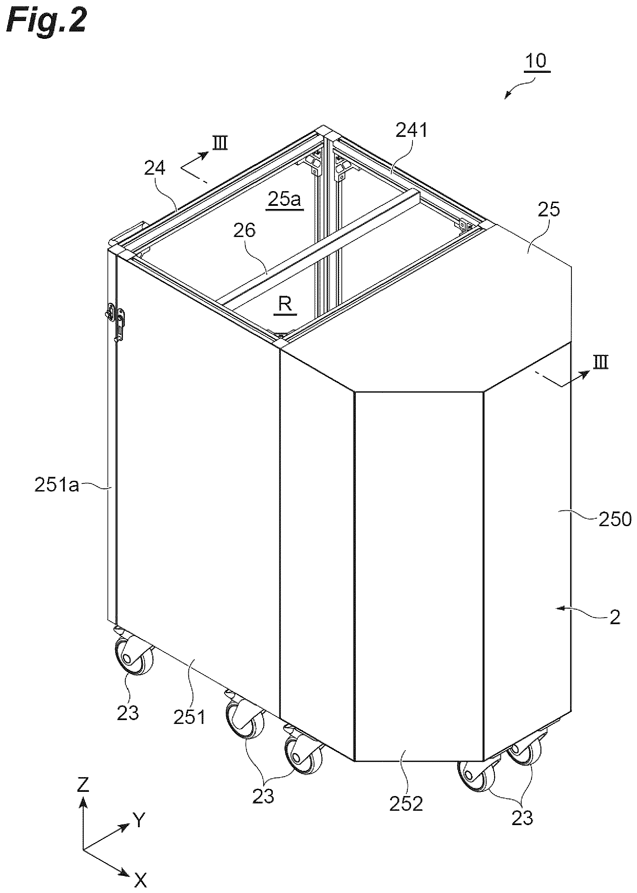

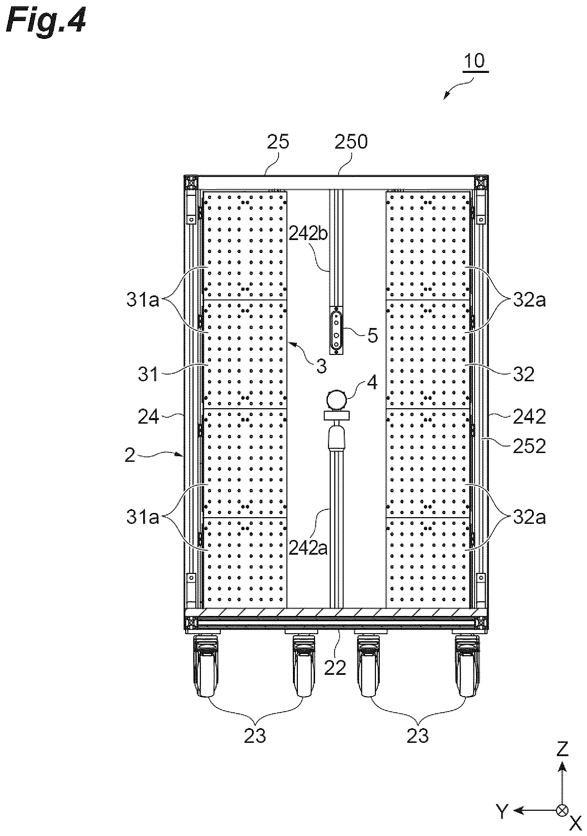

As illustrated in FIGS. 2, 3, 4, and 5, the device main body 10 includes a main body portion 2. The main body portion 2 includes a placement portion 21 and a support portion 22. In the main body portion 2, the placement portion 21 is a portion on which the subject S stands, and the support portion 22 is a portion disposed on one side in the X direction with respect to the placement portion 21. The main body portion 2 is positioned in a lower body L (that is, the portion from the lower abdomen to the toe) of the subject S in the imaging region R on the placement portion 21. A plurality of casters 23 are attached to lower surfaces of the placement portion 21 and the support portion 22. This enables smooth movement of the device main body 10.

The main body portion 2 further includes a frame 24 and a wall portion 25. The frame 24 is composed of a first frame 241 and a second frame 242. The first frame 241 is attached to the placement portion 21, and the second frame 242 is attached to the support portion 22. The wall portion 25 is composed of a first wall portion 251 and a second wall portion 252. The first wall portion 251 is attached to the placement portion 21 via the first frame 241, and the second wall portion 252 is attached to the support portion 22 via the second frame 242. The unit composed of the support portion 22, the second frame 242, and the second wall portion 252 is detachable from the unit composed of the placement portion 21, the first frame 241, and the first wall portion 251. As an example, the width of each unit in the X direction and the width of each unit in the Y direction are 70 cm or less. Accordingly, portability of each unit can be improved.

The wall portion 25 configures the housing 250 on the placement portion 21 and the support portion 22. The housing 250 defines the imaging region R on the placement portion 21. In the housing 250, an opening 25a is formed so as to face the placement portion 21 across the imaging region R. The waist portion of the subject S is positioned inside the opening 25a. That is, the opening 25a in which the subject S is disposed is formed in the wall portion 25. The inner surface of the housing 250 is, for example, black. Note that, in the first wall portion 251, a door is composed of a wall portion 251a on a side opposite to the second wall portion 252. Thus, the subject S can get on and off the placement portion 21.

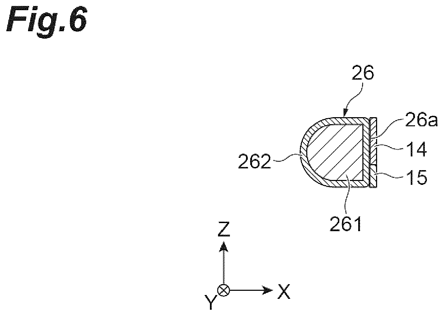

The main body portion 2 further includes a restricting portion 26. The restricting portion 26 is disposed along an upper end portion Ra of the imaging region R. The restricting portion 26 restricts the movement of the subject S to one side (support portion 22 side) in the X direction. In the present embodiment, the restricting portion 26 extends in the Y direction along the upper end portion Ra of the imaging region R and is stretched over the first frame 241 in a state of crossing the opening 25a. As illustrated in FIG. 6, the restricting portion 26 is composed of a bar material 261 and a cushion material 262. The bar material 261 is a core material stretched to the first frame 241. The cushion material 262 is an elastic material wound around the bar material 261.

As illustrated in FIGS. 2, 3, 4, and 5, the device main body 10 further includes an illumination unit 3, an imaging unit 4, and a distance sensor 5. The illumination unit 3, the imaging unit 4, and the distance sensor 5 are arranged on one side (support portion 22 side) in the X direction with respect to the imaging region R. The housing 250 accommodates the illumination unit 3, the imaging unit 4, and the distance sensor 5 on the support portion 22.

The illumination unit 3 irradiates the lower body L with excitation light. The illumination unit 3 includes a first light emitting region 31 and a second light emitting region 32 arranged in a state of being separated from each other in the Y direction. Each of the first light emitting region 31 and the second light emitting region 32 extends in the Z direction in a state of facing the imaging region R. The first light emitting region 31 and the second light emitting region 32 are arranged so as to be away from each other in the Y direction as approaching the imaging region R in the X direction. The first light emitting region 31 includes a plurality of first light emitting portions 31a, and the second light emitting region 32 includes a plurality of second light emitting portions 32a. The illumination unit 3 has a function of adjusting the light emission intensity of each of the first light emitting portions 31a and the light emission intensity of each of the second light emitting portions 32a. Each of the first light emitting portion 31a and the second light emitting portion 32a is composed of, for example, a plurality of LEDs that emit light having a center wavelength of 735 nm. The illumination unit 3 is attached to the second frame 242. That is, the illumination unit 3 is supported by the support portion 22 via the second frame 242.

The imaging unit 4 detects fluorescence emitted from the lower body L in response to irradiation with excitation light. The imaging unit 4 faces the imaging region R across a region between the first light emitting region 31 and the second light emitting region 32. The imaging unit 4 is disposed on one side (a side opposite to the imaging region R) in the X direction with respect to the illumination unit 3. That is, the illumination unit 3 is positioned between the imaging region R and the imaging unit 4 in the X direction. The imaging unit 4 is positioned in the center of the imaging region R when viewed from the X direction. In the present embodiment, the imaging unit 4 is attached to a bar material 242a which is a member extending upward from the support portion 22 side in the second frame 242. That is, the imaging unit 4 is supported by the support portion 22 via the second frame 242.

As an example, the imaging unit 4 is composed of a photodetector, a filter disposed in a preceding stage of the photodetector, a lens disposed in a preceding stage of the filter, and the like. As the photodetector, an area sensor (for example, CCD and CMOS) having sensitivity to fluorescence (for example, light in the near-infrared region) is used. As the filter, a vapor deposition filter that selectively transmits fluorescence is used. The filter may include an absorption filter to remove the influence of oblique light. As the lens, a wide-angle lens is used so that an image of the entire lower body L can be captured.

The distance sensor 5 measures a distance to the lower body L positioned in the imaging region R. The distance sensor 5 may be, for example, an ultrasonic distance sensor or an optical distance sensor such as a triangulation method or a ToF method. The distance sensor 5 faces the imaging region R across a region between the first light emitting region 31 and the second light emitting region 32. The distance sensor 5 is disposed on one side (a side opposite to the imaging region R) in the X direction with respect to the illumination unit 3. The distance sensor 5 is positioned in the center of the imaging region R in the Y direction when viewed from the X direction. In the present embodiment, the distance sensor 5 is attached to a bar material 242b which is a member extending downward from the support portion 22 side in the second frame 242. That is, the distance sensor 5 is supported by the support portion 22 via the second frame 242. Note that, based on the distance measured by the distance sensor 5, the light emission intensity of the illumination unit 3 may be adjusted so that the intensity of the excitation light with which the lower body L is irradiated becomes uniform. Such adjustment of the light emission intensity of the illumination unit 3 may be performed based on the intensity of the excitation light detected by a photodetector provided separately.

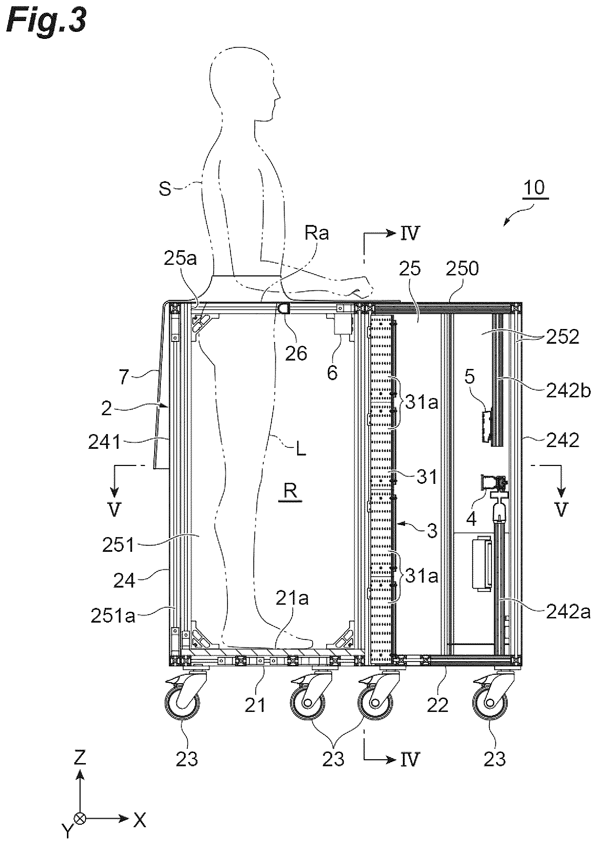

As illustrated in FIG. 3, the device main body 10 includes a camera 6. The camera 6 faces the placement portion 21 and is attached to the first frame 241 so as not to enter the field of view of the imaging unit 4. The housing 250 accommodates the camera 6 on the placement portion 21. The space in the housing 250 is set as a dark room by a cover 7 that covers the opening 25a in a state of being attached to the subject S. Since the camera 6 captures an image of the feet of the subject S in the space in the housing 250 that is set as a dark room, the camera 6 may be composed of an illumination device and an image capturing device or may be configured only with the image capturing device such as an infrared camera. The image acquired by the camera 6 is displayed by the display unit 12 to be shown to the subject S. Note that the camera 6 may be attached to the restricting portion 26 or may be attached to the second frame 242. When the camera 6 is composed of an illumination device and an imaging device, the illumination device may be separated from the image capturing device.

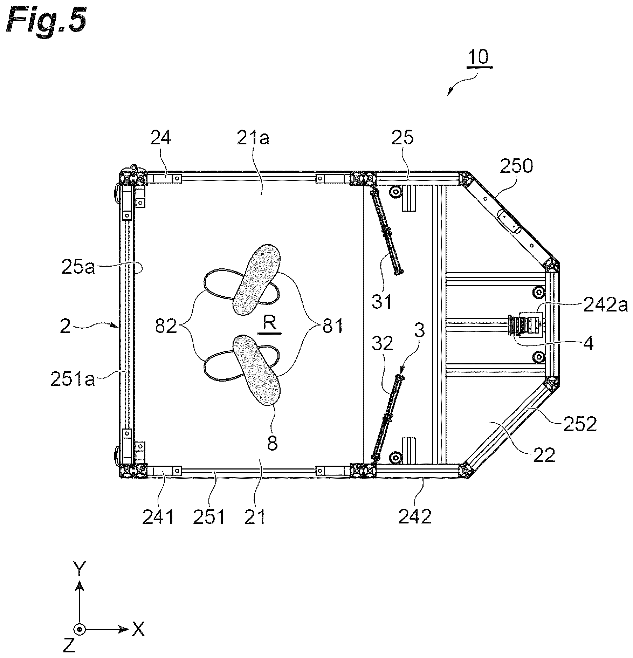

As illustrated in FIG. 5, the placement portion 21 is provided with a positioning portion 8 that indicates the standing position of the subject S. In the present embodiment, the positioning portion 8 includes a foot rest 81 in a state where the subject S faces the imaging unit 4 side, and a foot rest 82 in a state where the subject S faces the side opposite to the imaging unit 4. Each of the foot rests 81 and 82 is a mark formed on the placement surface 21a of the placement portion 21. The image of the positioning portion 8 is captured by the camera 6.

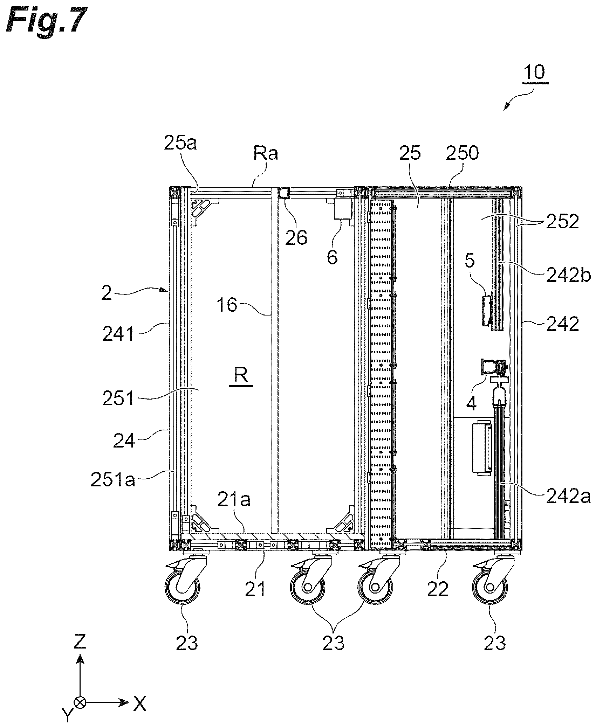

As illustrated in FIG. 6, a first plate 14 and a scale 15 are attached to an external surface 26a of the restricting portion 26 on the imaging unit 4 side. The first plate 14 is a plate for luminance calibration. As an example, the first plate 14 is composed of a plurality of types of members (for example, a cloth and an acrylic plate) having different intensities of fluorescence emitted in response to irradiation with excitation light. The scale 15 is a member having a gradation indicating a length. As illustrated in FIG. 7, a second plate 16 is detachable from the main body portion 2. The second plate 16 is disposed on the main body portion 2 so as to be positioned in the imaging region R in a state of being in contact with the restricting portion 26 from the side opposite to the imaging unit 4. The second plate 16 is a plate for luminance calibration and is, for example, an acrylic plate.

In the lymphatic system examination device 1 configured as described above, the control unit 11 is electrically connected to each of the illumination unit 3, the imaging unit 4, the distance sensor 5, the camera 6, the display unit 12, and the input unit 13. The control unit 11 controls the illumination unit 3 to emit the excitation light as a flash in accordance with the image capturing timing of the imaging unit 4. As a result, it is possible to suppress a change in the light amount of the excitation light applied to the lower body L at the time of capturing an image of the lower body L. The control unit 11 generates a fluorescent image of the lower body L based on the fluorescence detected by the imaging unit 4, and controls the display unit 12 to display the fluorescent image of the lower body L. The control unit 11 stores the fluorescent image of the lower body L generated in the past for the subject S and can control the display unit 12 so that the fluorescent image of the lower body L generated in the past and the fluorescent image of the lower body L generated newly are displayed side by side. In addition, the control unit 11 can control the display unit 12 so that the visible-light image of the lower body L and the fluorescent image of the lower body L are displayed side by side. Furthermore, the control unit 11 can control the display unit 12 so that a part of at least one image of the lower body L (visible-light image, fluorescent image) is enlarged based on the instruction input to the control unit 11 via the input unit 13.

In the present embodiment, the control unit 11 controls at least one of the illumination unit 3 and the imaging unit 4 so as to change at least one of the light emission time of the illumination unit 3 and the exposure time of the imaging unit 4, thereby generating a plurality of types of fluorescent images as the fluorescent image of the lower body L and controlling the display unit 12 so that the plurality of types of fluorescent images are displayed side by side. That is, in the lymphatic system examination device 1, a lymphatic system examination method including a step of generating a plurality of types of fluorescent images as the fluorescent image of the lower body L by controlling at least one of the illumination unit 3 and the imaging unit 4 so that at least one of the light emission time of the illumination unit 3 and the exposure time of the imaging unit 4 is changed (that is, the exposure amount in the imaging unit 4 is changed), and a step of controlling the display unit 12 so that the plurality of types of fluorescent images are displayed side by side is performed.

In the lymphatic system examination method, the control unit 11 generates each of the plurality of types of fluorescent images in a time of one second or less (that is, the time is one second or less after the image capturing instruction is input to the control unit 11 via the input unit 13). Note that the exposure amount in the imaging unit 4 may be changed by changing at least one of the light emission time of the illumination unit 3, the light emission intensity of the illumination unit 3, the exposure time of the imaging unit 4, the sensor gain of the imaging unit 4, and the lens diaphragm of the imaging unit 4.

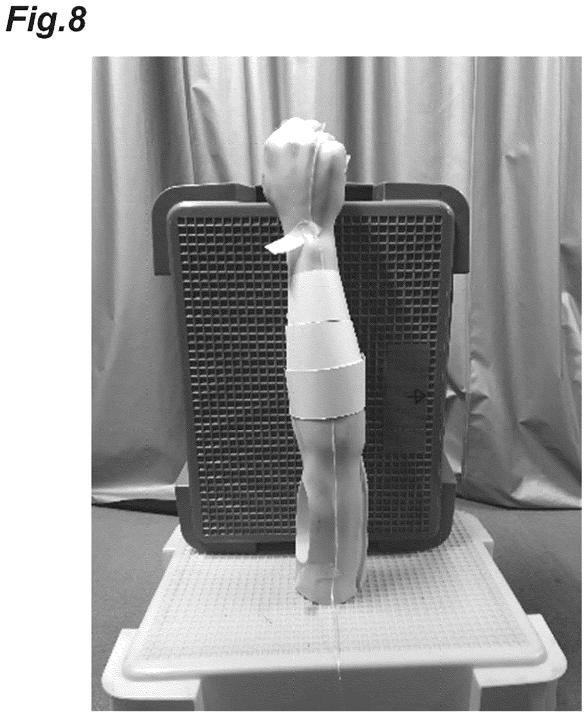

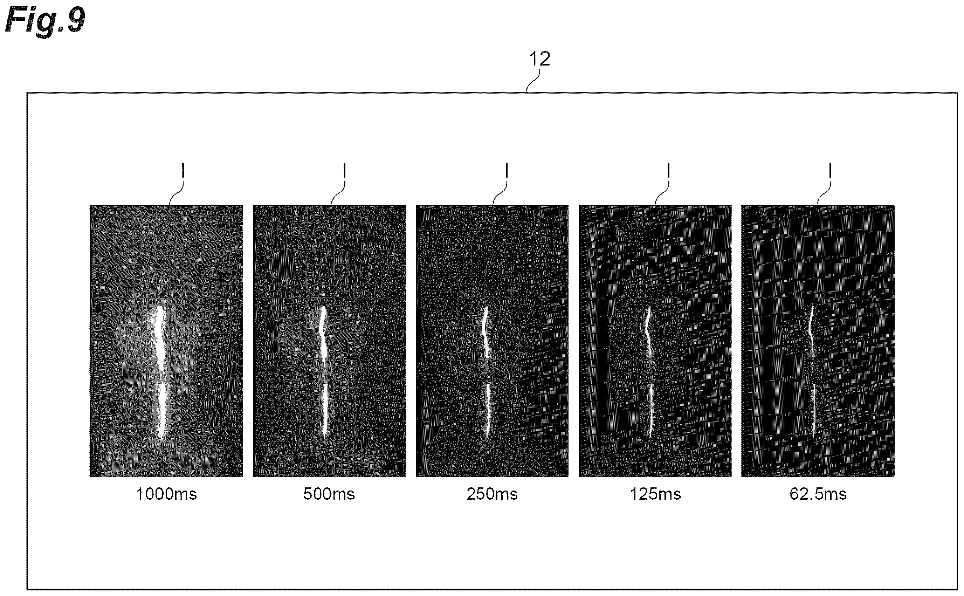

FIG. 8 is a diagram illustrating a visible-light image of the object, and FIG. 9 is a diagram illustrating the display unit 12 on which a plurality of types of fluorescent images I for the object illustrated in FIG. 8 are displayed. The object illustrated in FIG. 8 is obtained by winding white paper around an arm model to which a phosphor extending in the vertical direction is adhered. The white paper covers the phosphor in a single, double, and triple state in the order from the top. The plurality of types of fluorescent images I illustrated in FIG. 9 are images generated with the light emission time of the illumination unit 3 and the exposure time of the imaging unit 4 set to 1000 ms, 500 ms, 250 ms, 125 ms, and 62.5 ms. When the light emission time of the illumination unit 3 and the exposure time of the imaging unit 4 are 500 ms or more, the portion of the single white paper is observed to be considerably thicker than the actual phosphor due to the scattering of the fluorescence. When the light emission time of the illumination unit 3 and the exposure time of the imaging unit 4 are 125 ms or more, the phosphor can be visually recognized in the portion of the double white paper. When the light emission time of the illumination unit 3 and the exposure time of the imaging unit 4 are 500 ms or more, the phosphor can be visually recognized in the portion of the triple white paper. From the above, it can be seen that the state of the lymphatic system in the lower body L can be appropriately grasped by adjusting at least one of the light emission time of the illumination unit 3 and the exposure time of the imaging unit 4.

As described above, in the lymphatic system examination device 1, each of the first light emitting region 31 and the second light emitting region 32 in the illumination unit 3 extends in the Z direction in a state of facing the imaging region R, and the imaging unit 4 faces the imaging region R across a region between the first light emitting region 31 and the second light emitting region 32. As a result, the lower body L of the subject S positioned in the imaging region R can be uniformly irradiated with the excitation light, and the fluorescence emitted from the lower body L in response to the uniform irradiation of the excitation light can be detected. Therefore, according to the lymphatic system examination device 1, it is possible to acquire a fluorescent image of the lower body L appropriate for examination of the lymphatic system in the lower body L of the subject S.

In the lymphatic system examination device 1, the imaging unit 4 is positioned at the center of the imaging region R when viewed from the X direction. This makes it possible to suppress occurrence of distortion in the acquired fluorescent image.

In the lymphatic system examination device 1, the illumination unit 3 is positioned between the imaging region R and the imaging unit 4 in the X direction. As a result, the lower body L of the subject S positioned in the imaging region R can be more uniformly irradiated with the excitation light.

In the lymphatic system examination device 1, the illumination unit 3 has a function of adjusting the light emission intensity of each of the first light emitting portions 31a and the light emission intensity of each of the second light emitting portions 32a. As a result, in response to the shape and the like of the lower body L of the subject S positioned in the imaging region R, the lower body L can be uniformly irradiated with the excitation light.

In the lymphatic system examination device 1, the first light emitting region 31 and the second light emitting region 32 are arranged so as to be away from each other in the Y direction as approaching the imaging region R in the X direction. As a result, the lower body L of the subject S positioned in the imaging region R can be more uniformly irradiated with the excitation light.

In addition, in the lymphatic system examination device 1, the illumination unit 3 and the imaging unit 4 are arranged on one side in the X direction with respect to the imaging region R where the lower body L of the subject S is positioned, and the movement of the subject S to one side in the X direction is restricted by the restricting portion 26 arranged along the upper end portion Ra of the imaging region R. As a result, the distance between the illumination unit 3 and the lower body L of the subject S and the distance between the imaging unit 4 and the lower body L of the subject S can be maintained constant, and shaking of the lower body L of the subject S can be suppressed. Therefore, according to the lymphatic system examination device 1, it is possible to acquire a fluorescent image of the lower body L appropriate for examination of the lymphatic system in the lower body L of the subject S.

In the lymphatic system examination device 1, the restricting portion 26 extends in the Y direction, and the first plate 14 for luminance calibration is disposed on the external surface 26a of the restricting portion 26 on the imaging unit 4 side. As a result, the luminance of the fluorescent image can be adjusted for each acquired fluorescent image.

In the lymphatic system examination device 1, the restricting portion 26 extends in the Y direction, and the scale 15 is disposed on the external surface 26a of the restricting portion 26 on the imaging unit 4 side. As a result, the size of each part of the lower body L can be grasped from the acquired fluorescent image.

In the lymphatic system examination device 1, the second plate 16 for luminance calibration is disposed on the main body portion 2 so as to be positioned in the imaging region R in a state of being in contact with the restricting portion 26 from the side opposite to the imaging unit 4. As a result, the luminance of the fluorescent image can be adjusted for each lymphatic system examination device 1.

In the lymphatic system examination device 1, the distance sensor 5 is disposed on one side in the X direction with respect to the imaging region R. As a result, the position of the lower body L of the subject S in the X direction can be accurately grasped.

In addition, in the lymphatic system examination device 1 (and the above-described lymphatic system examination method), by changing at least one of the light emission time of the illumination unit 3 and the exposure time of the imaging unit 4, a plurality of types of fluorescent images are generated as the fluorescent image of the lower body L of the subject S, and the plurality of types of fluorescent images are displayed side by side. Although the intensity of fluorescence changes in response to the state of the lymphatic system in the lower body L, the state of the lymphatic system in the lower body L can be appropriately grasped from the plurality of types of fluorescent images generated and displayed as described above. Therefore, according to the lymphatic system examination device 1 (and the above-described lymphatic system examination method), the examination of the lymphatic system in the lower body L of the subject S can be appropriately performed.

In the lymphatic system examination device 1, the control unit 11 generates each of the plurality of types of fluorescent images during the time of one second or less. As a result, it is possible to acquire a plurality of types of fluorescent images while shaking of the lower body L of the subject S is suppressed.

In the lymphatic system examination device 1, the control unit 11 controls the illumination unit 3 so as to emit the excitation light as a flash. As a result, it is possible to acquire a plurality of types of fluorescent images while change of the light amount of the excitation light applied to the lower body L of the subject S is suppressed.

In lymphatic system examination device 1, the control unit 11 stores the fluorescent image of the lower body L generated in the past for the subject S and controls the display unit 12 so that the fluorescent image of the lower body L generated in the past and the fluorescent image of the lower body L generated newly are displayed side by side. This makes it possible to appropriately grasp the temporal change in the state of the lymphatic system in the lower body L of the subject S.

In the lymphatic system examination device 1, the positioning portion 8 indicating the standing position of the subject S is provided in the placement portion 21 on which the subject S stands. As a result, the distance between the illumination unit 3 and the lower body L of the subject S and the distance between the imaging unit 4 and the lower body L of the subject S can be maintained constant, and the direction of both feet of the subject S with respect to the illumination unit 3 and the imaging unit 4 can be matched to a predetermined direction. Therefore, according to the lymphatic system examination device 1, it is possible to acquire a fluorescent image of the lower body L appropriate for examination of the lymphatic system in the lower body L of the subject S.

In the lymphatic system examination device 1, the positioning portion 8 includes the foot rest 81 in a state where the subject S faces the imaging unit 4 side, and the foot rest 82 in a state where the subject S faces the side opposite to the imaging unit 4. As a result, in each of the state in which the subject S faces the imaging unit 4 side and the state in which the subject S faces the side opposite to the imaging unit 4, the direction of both feet of the subject S with respect to the illumination unit 3 and the imaging unit 4 can be matched to a predetermined direction.

In the lymphatic system examination device 1, the wall portion 25 configures the housing 250 that defines the imaging region R and accommodates the illumination unit 3 and the imaging unit 4, and the opening 25a in which the subject S is disposed is formed in the wall portion 25. As a result, it is possible to acquire the fluorescent image of the lower body L of the subject S while the influence of the disturbance light is suppressed.

In the lymphatic system examination device 1, the cover 7 attached to the subject S covers the opening 25a. As a result, it is possible to acquire the fluorescent image of the lower body L of the subject S while the influence of the disturbance light is more reliably suppressed.

In the lymphatic system examination device 1, the support portion 22 and the second wall portion 252 are detachable from the placement portion 21 and the first wall portion 251. Accordingly, portability of the lymphatic system examination device 1 can be improved.

In the lymphatic system examination device 1, the image of the positioning portion 8 is captured by the camera 6. As a result, even when it is difficult for the subject S to directly visually recognize the positioning portion 8, the subject S can match the standing position with the positioning portion 8 while visually recognizing the image acquired by the camera 6.

Here, correction processing of the fluorescence image (hereinafter, it is simply referred to as a "fluorescence image of the subject S") of the lower body L of the subject S based on the excitation light image (hereinafter, simply referred to as an "excitation light image of the subject S") of the lower body L of the subject S (the lymphatic system examination method performed in the lymphatic system examination device 1) is described.

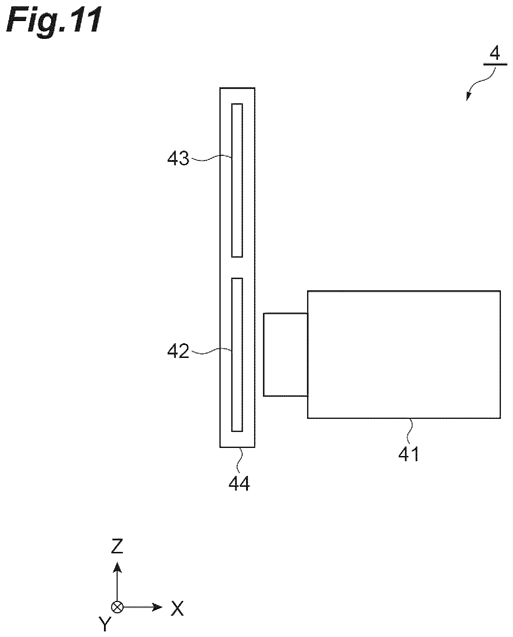

As illustrated in FIG. 11, the imaging unit 4 used for the correction processing includes a camera 41, a light transmitting member 42, a filter 43, and a holding member 44. The camera 41 is sensitive to excitation light (hereinafter, simply referred to as "excitation light") emitted from the illumination unit 3 and fluorescence (hereinafter, simply referred to as "fluorescence") emitted from the lower body L. For example, an area sensor such as a CCD or a CMOS is used as the camera 41. The light transmitting member 42 transmits the excitation light and the fluorescence. The light transmitting member 42 is, for example, a glass plate. The filter 43 cuts off the excitation light to selectively transmit the fluorescence. The filter 43 is, for example, a longpass filter or a bandpass filter.

The holding member 44 holds the light transmitting member 42 and the filter 43. The holding member 44 disposes the light transmitting member 42 or the filter 43 on the preceding stage (the imaging region R side) of the camera 41. The holding member 44 is, for example, a wheel that rotates about an axis parallel to the X direction as a center line, or a slider that reciprocates along a predetermined direction perpendicular to the X direction. In the present embodiment, the holding member 44 is controlled by the control unit 11 so that the light transmitting member 42 or the filter 43 is disposed at the preceding stage of the camera 41.

Note that the light transmitting member 42 has a thickness (ideally, the thickness that causes that the difference between the optical path lengths to become 0) so that the optical path length of the excitation light transmitted through the light transmitting member 42 and incident on the camera 41 approaches the optical path length of the fluorescence transmitted through the filter 43 and incident on the camera 41. However, the imaging unit 4 may not include the light transmitting member 42. In addition, although not illustrated, a wide-angle lens is disposed at the preceding stage of the light transmitting member 42 or the filter 43 disposed at the preceding stage of the camera 41 so that the image of the entire lower body L can be captured. The wide-angle lens may be provided in the camera 41 so as to be disposed between the light transmitting member 42 or the filter 43 disposed in the preceding stage of the camera 41 and the area sensor of the camera 41.

In a state where the imaging unit 4 configured as described above is provided in the device main body 10, the control unit 11 performs "processing of generating filter correction data" and "processing of generating a fluorescence image of the subject S" as the correction processing of the fluorescence image of the subject S based on the excitation light image of the subject S. The "processing of generating the filter correction data" is performed at the timing of calibration of the lymphatic system examination device 1. The "generation processing of the fluorescence image of the subject S" is performed at the timing of acquiring the fluorescence image of the subject S.

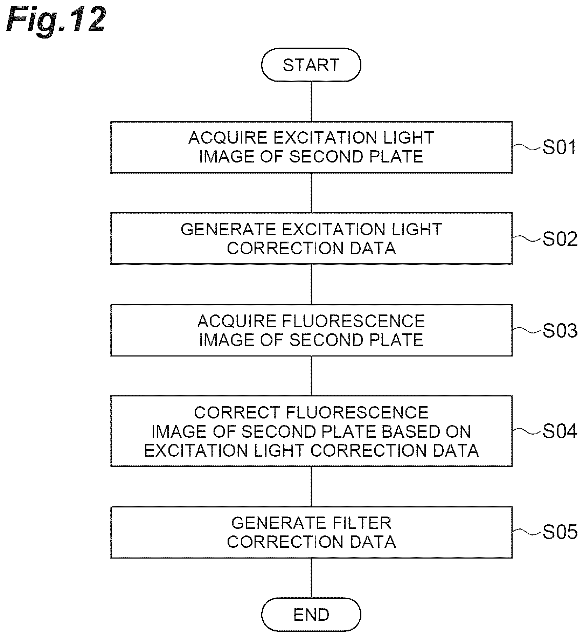

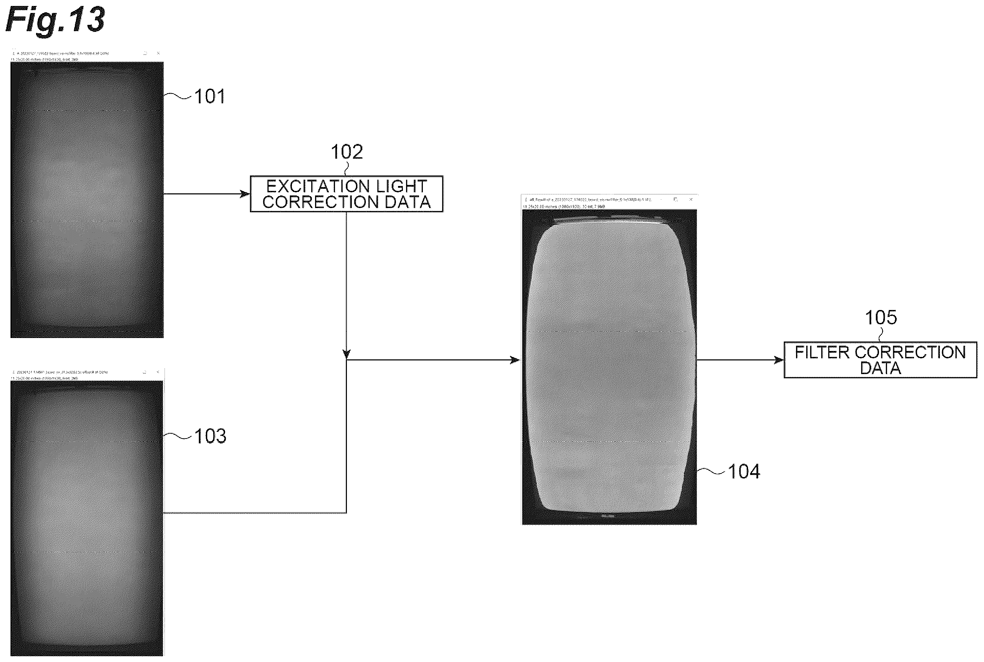

First, the "processing of generating the filter correction data" is described with reference to FIGS. 12 and 13. First, the second plate (plate for luminance calibration) 16 is disposed on the main body portion 2 so as to be positioned in the imaging region R in a state of being in contact with the restricting portion 26 from the side opposite to the imaging unit 4 (see FIG. 7). In a state where the second plate is positioned in the imaging region R, the light transmitting member 42 in the imaging unit 4 is disposed at the preceding stage of the camera 41 (see FIG. 11). Then, the illumination unit 3 irradiates the second plate 16 with excitation light, and the imaging unit 4 acquires an excitation light image 101 of the second plate 16 (S01). The excitation light image 101 is an image of the second plate 16 obtained with the excitation light (excitation light that is reflected by the second plate 16, transmitted through the light transmitting member 42, and incident onto the camera 41). The fluorescence emitted from the second plate 16 in response to the irradiation of the excitation light is also transmitted through the light transmitting member 42 and incident onto the camera 41 together with the excitation light. However, since the intensity of the fluorescence is much lower than the intensity of the excitation light, the influence of the fluorescence can be substantially ignored in the excitation light image 101. Subsequently, the control unit 11 generates excitation light correction data 102 based on the excitation light image 101 of the second plate 16 (S02). The excitation light correction data 102 is data for correcting (uniformizing) "unevenness of the excitation light image of the second plate 16" caused by "non-uniformity of the irradiation intensity of the excitation light in the second plate 16" in the excitation light image 101 and is data for uniformizing the luminance value of each pixel that configures the excitation light image of the second plate 16.

Subsequently, in a state where the second plate is positioned in the imaging region R (see FIG. 7), the filter 43 is disposed in a preceding stage of the camera 41 in the imaging unit 4 (see FIG. 11). Then, the illumination unit 3 irradiates the second plate 16 with excitation light, and the imaging unit 4 acquires a fluorescence image 103 of the second plate 16 (S03). The fluorescence image 103 is an image of the second plate 16 by fluorescence (fluorescence emitted from the second plate 16 in response to irradiation of excitation light, the fluorescence transmitting through the filter 43 and being incident onto the camera 41). Subsequently, the control unit 11 corrects the fluorescence image 103 of the second plate 16 based on the excitation light correction data 102 to generate a corrected fluorescence image 104 of the second plate 16 (S04). Subsequently, the control unit 11 generates filter correction data 105 based on the corrected second plate 16 of the fluorescence image 104 (S05). The filter correction data 105 is data for correcting (uniformizing) "unevenness of the fluorescent image of the second plate 16" caused by "angular dependence of transmission wavelength in the filter 43" in the fluorescence image 104 and is data for uniformizing the luminance value of each pixel that configures the fluorescent image of the second plate 16. The control unit 11 stores the generated filter correction data 105.

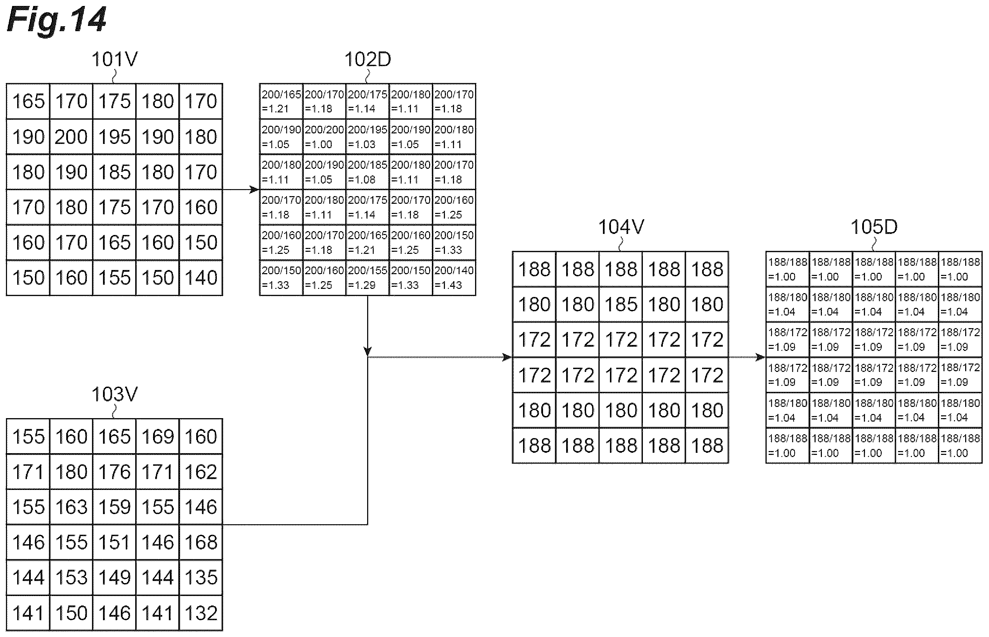

A specific example of the above-described "processing of generating the filter correction data" is described with reference to FIG. 14. As illustrated in FIG. 14, in the specific example, it is assumed that an excitation light image 101V is acquired as the excitation light image 101 of the second plate 16, and a fluorescence image 103V is acquired as the fluorescence image 103 of the second plate 16. Each of the excitation light image 101V and the fluorescence image 103V is composed of a plurality of pixels arranged in a matrix. In each of the excitation light image 101V and the fluorescence image 103V, a numerical value described in each pixel indicates a luminance value.

In this specific example, as the excitation light correction data 102, excitation light correction data 102D is generated based on the excitation light image 101V. The excitation light correction data 102D is composed of a plurality of coefficients. Each coefficient of the excitation light correction data 102D corresponds to each pixel of the excitation light image 101V. The coefficient corresponding to a certain pixel is a value obtained by dividing the maximum value ("200" in this specific example) of the luminance values of all the pixels by the luminance value of the pixel. Here, a pixel having a luminance value less than the predetermined threshold ("30" in this specific example) is regarded as a pixel that configures the background of the second plate 16, and a coefficient corresponding to the pixel is set to "1".

In this specific example, as the fluorescence image 104 of the second plate 16, a fluorescence image 104V is generated by correcting the fluorescence image 103V based on the excitation light correction data 102D. The fluorescence image 104V is generated by multiplying each pixel of the fluorescence image 103V by each coefficient of the excitation light correction data 102D.

In this specific example, as the filter correction data 105, filter correction data 105D is generated based on the fluorescence image 104V. The filter correction data 105D is composed of a plurality of coefficients. Each coefficient of the filter correction data 105 corresponds to each pixel of the fluorescence image 104V. The coefficient corresponding to a certain pixel is a value obtained by dividing the maximum value ("188" in this specific example) of the luminance values of all the pixels by the luminance value of the pixel. Here, a pixel having a luminance value less than the predetermined threshold ("30" in this specific example) is also regarded as a pixel that configures the background of the second plate 16, and a coefficient corresponding to the pixel is set to "1".

Note that the luminance value used as a reference when each of the excitation light correction data 102D and the filter correction data 105D is generated is not limited to the maximum value of the luminance values of all the pixels and may be a value of the top n% of the luminance value distribution, an average luminance value, or the like in order to exclude spike noise. In order to remove the local coefficient fluctuation in each of the excitation light correction data 102D and the filter correction data 105D, a two-dimensional filter such as a smoothing filter or a Gaussian filter may be applied when each of the excitation light correction data 102D and the filter correction data 105D is calculated.

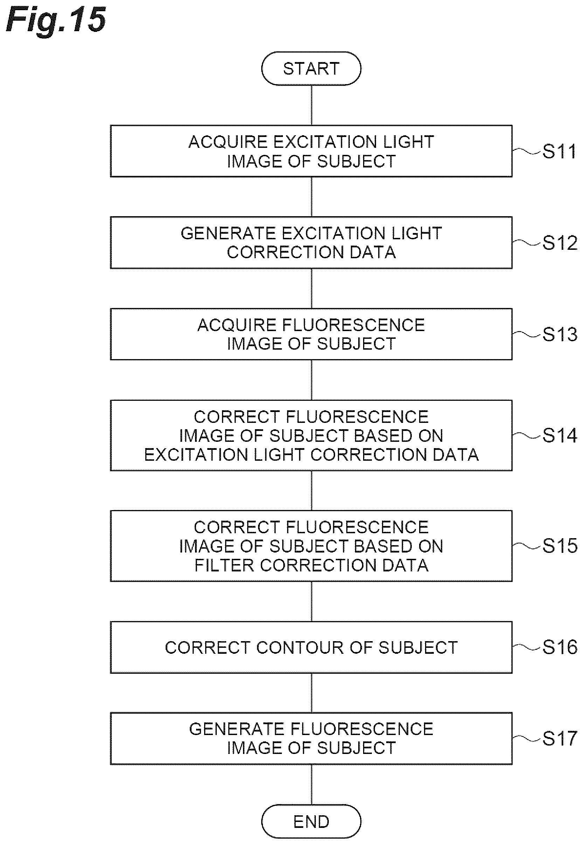

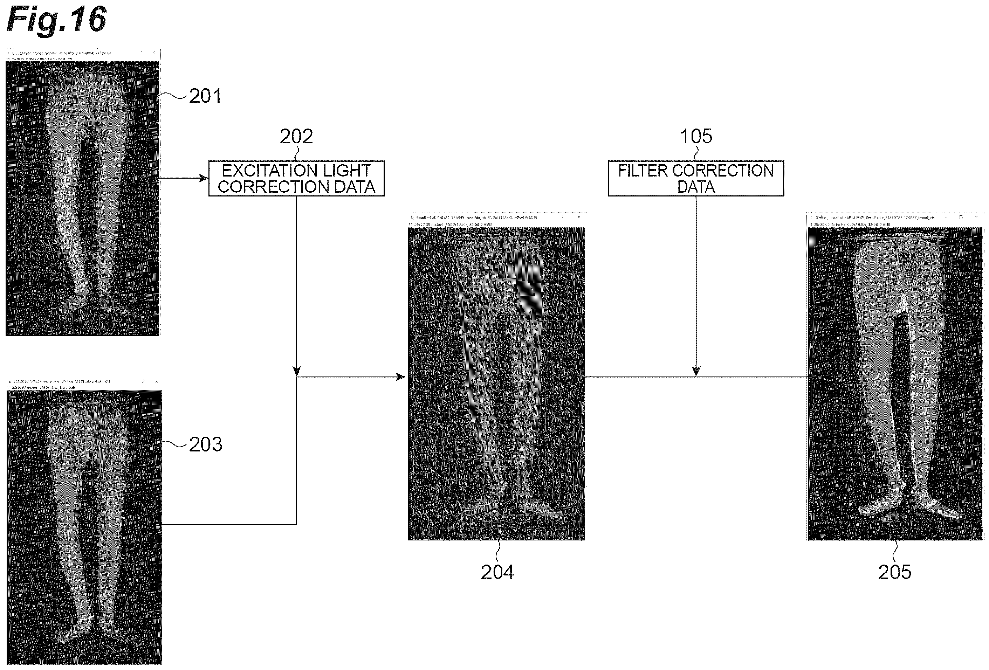

Next, the "generation processing of the fluorescence image of the subject S" is described with reference to FIGS. 15 and 16. First, the subject S stands on the placement portion 21 so that the lower body L is positioned in the imaging region R (see FIG. 3). In a state where the lower body L is positioned in the imaging region R, the light transmitting member 42 in the imaging unit 4 is disposed at the preceding stage of the camera 41 (see FIG. 11). Then, the illumination unit 3 irradiates the lower body L of the subject S with the excitation light, and the imaging unit 4 acquires an excitation light image 201 of the subject S (a step of acquiring an excitation light image of the lower body, S11). The excitation light image 201 is an image of the lower body L obtained with the excitation light (excitation light that is reflected by the lower body L of the subject S, transmitted through the light transmitting member 42, and incident onto the camera 41). The fluorescence emitted from the lower body L of the subject S in response to the irradiation of the excitation light is also transmitted through the light transmitting member 42 and incident onto the camera 41 together with the excitation light. However, since the intensity of the fluorescence is much lower than the intensity of the excitation light, the influence of the fluorescence can be substantially ignored in the excitation light image 201. Subsequently, the control unit 11 generates excitation light correction data 202 based on the excitation light image 201 of the subject S (S12). The excitation light correction data 202 is data for correcting (uniformizing) "unevenness of the excitation light image of the lower body L" caused by "non-uniformity of the irradiation intensity of the excitation light in the lower body L" in the excitation light image 201 and is data for uniformizing the luminance value of each pixel that configures the excitation light image of the lower body L.

Subsequently, in a state where the lower body L is positioned in the imaging region R (see FIG. 3), the filter 43 is disposed in a preceding stage of the camera 41 in the imaging unit 4 (see FIG. 11). Then, the illumination unit 3 irradiates the lower body L of the subject S with the excitation light, and the imaging unit 4 acquires a fluorescence image 203 of the subject S (a step of acquiring a fluorescence image of the lower body, S13). The fluorescence image 203 is an image of the lower body L by fluorescence (fluorescence emitted from the lower body L of the subject S in response to irradiation of excitation light, the fluorescence transmitting through the filter 43 and being incident onto the camera 41). Subsequently, the control unit 11 corrects the fluorescence image 203 of the subject S based on the excitation light correction data 202 to generate a corrected fluorescence image 204 of the subject S (a step of correcting a fluorescence image of the lower body, S14). Subsequently, the control unit 11 corrects the fluorescence image 204 of the subject S based on the filter correction data 105 stored in advance, thereby generating a further corrected fluorescence image 205 of the subject S (S15). Note that, for the fluorescence image 203 of the subject S, the correction based on either the excitation light correction data 202 and the filter correction data 105 may be performed first, or the correction based on both the data may be simultaneously performed.

Subsequently, the control unit 11 corrects the contour of the fluorescent image of the lower body L in the fluorescence image 205 of the subject S (S16) and generates the fluorescence image of the subject S (S17). The control unit 11 causes the display unit 12 to display the generated fluorescence image of the subject S. In some cases, the control unit 11 generates a plurality of images arranged in time series as the fluorescence image of the subject S and controls the display unit 12 so that the plurality of images is continuously displayed. The contour of the fluorescent image of the lower body L is corrected in the fluorescence image 205 of the subject S, because the contour of the fluorescent image of the lower body L may be emphasized by the correction based on the excitation light correction data 202. For the correction of the contour, for example, a blurring filter is used.

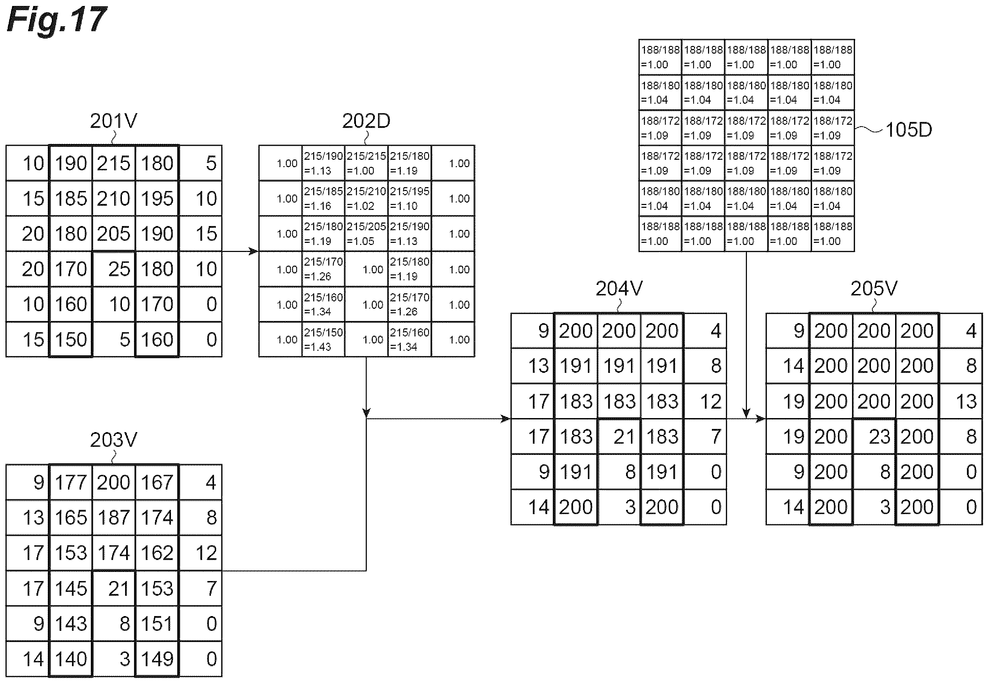

A specific example of the above-described "generation processing of the fluorescence image of the subject S" is described with reference to FIG. 17. As illustrated in FIG. 17, in the specific example, it is assumed that an excitation light image 201V is acquired as the excitation light image 201 of the subject S, and a fluorescence image 203V is acquired as the fluorescence image 203 of the subject S. Each of the excitation light image 201V and the fluorescence image 203V is composed of a plurality of pixels arranged in a matrix. In each of the excitation light image 201V and the fluorescence image 203V, a numerical value described in each pixel indicates a luminance value.

In this specific example, as the excitation light correction data 202, excitation light correction data 202D is generated based on the excitation light image 201V. The excitation light correction data 202D is composed of a plurality of coefficients. Each coefficient of the excitation light correction data 202D corresponds to each pixel of the excitation light image 201V. The coefficient corresponding to a certain pixel is a value obtained by dividing the maximum value ("215" in this specific example) of the luminance values of all the pixels by the luminance value of the pixel. Here, a pixel having a luminance value less than the predetermined threshold ("30" in this specific example) is regarded as a pixel that configures the background of the lower body L, and a coefficient corresponding to the pixel is set to "1".

In this specific example, as the fluorescence image 204 of the subject S, a fluorescence image 204V is generated by correcting the fluorescence image 203V based on the excitation light correction data 202D. The fluorescence image 204V is generated by multiplying each pixel of the fluorescence image 203V by each coefficient of the excitation light correction data 202D. Further, in this specific example, as the fluorescence image 205 of the subject S, a fluorescence image 205V is generated by correcting the fluorescence image 204V based on the filter correction data 105D illustrated in FIG. 14. The fluorescence image 205V is generated by multiplying each pixel of the fluorescence image 204V by each coefficient of the filter correction data 105D.

Note that the luminance value used as a reference when the excitation light correction data 202D is generated is not limited to the maximum value of the luminance values of all the pixels and may be a value of the top n% of the luminance value distribution, an average luminance value, or the like in order to exclude spike noise. Furthermore, in order to remove a local coefficient fluctuation in the excitation light correction data 202D, a two-dimensional filter such as a smoothing filter or a Gaussian filter may be applied when the excitation light correction data 202D is calculated.

As described above, in the lymphatic system examination device 1 (the lymphatic system examination method performed in the lymphatic system examination device 1), the excitation light image 201 of the subject S and the fluorescence image 203 of the subject S are acquired, the excitation light correction data 202 is generated based on the excitation light image 201 of the subject S, and fluorescence image 203 of the subject S is corrected based on the excitation light correction data 202. As a result, it is possible to suppress the occurrence of unevenness in the fluorescent image of the lower body L due to the non-uniformity of the irradiation intensity of the excitation light in the lower body L. Therefore, according to the lymphatic system examination device 1 (the lymphatic system examination method performed in the lymphatic system examination device 1), it is possible to acquire a fluorescent image of the lower body L appropriate for examination of the lymphatic system in the lower body L of the subject S.

In the lymphatic system examination device 1, the excitation light image 101 of the second plate 16 and the fluorescence image 103 of the second plate 16 are acquired, the filter correction data 105 is generated based on the excitation light image 101 of the second plate 16 and the fluorescence image 103 of the second plate 16, and the fluorescence image 203 of the subject S is corrected based on the excitation light correction data 202 and the filter correction data 105. As a result, it is possible to suppress the occurrence of unevenness in the fluorescent image of the lower body L due to the angular dependence of the transmission wavelength in the filter 43.

In the lymphatic system examination device 1, the contour of the fluorescent image of the lower body L is corrected in the fluorescence image 205 of the subject S. As a result, it is possible to suppress the emphasis of the contour of the fluorescent image of the lower body L by the correction based on the excitation light correction data 202.

In the lymphatic system examination device 1, a plurality of images arranged in time series is generated as the corrected fluorescence image of the subject S, and the plurality of images is continuously displayed by the display unit 12. As a result, the fluorescent image of the lower body L can be continuously grasped in time series.

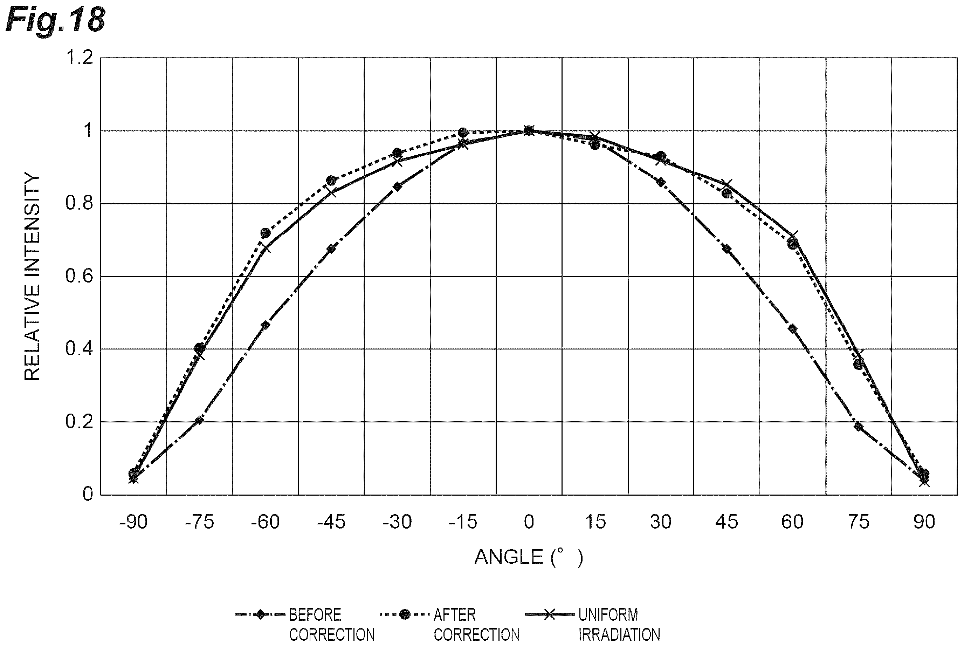

FIG. 18 is a diagram illustrating the effect of processing of generating the fluorescence image of the subject. In the verification in which the results illustrated in FIG. 18 is obtained, a capillary tube including indocyanine green and having an inner diameter of 0.4 mm (hereinafter, simply referred to as a "capillary tube") is prepared as an image capturing target. Then, in the above-described lymphatic system examination device 1, the capillary tube is disposed so as to extend in the Z direction at each angle position on the cylindrical surface having the "center line passing through the center of the imaging region R and extending in the Z direction", and the excitation light image of the capillary tube and the fluorescence image of the capillary tube are acquired. Note that 0° is a position of the front surface of the imaging unit 4, a positive angle is a position on the right side as viewed from the imaging unit 4 side, and a negative angle is a position on the left side as viewed from the imaging unit 4 side.

In FIG. 18, the result "before correction" is the relative intensity of the luminance value of the fluorescent image of the capillary tube when the fluorescence image of the capillary tube is not corrected. The result of "after correction" is the relative intensity of the luminance value of the fluorescent image of the capillary tube when the excitation light correction data is generated based on the excitation light image of the capillary tube and the fluorescence image of the capillary tube is corrected based on the excitation light correction data. The result of "uniform irradiation" is a result acquired under the condition that the excitation light panel is disposed at the position of the front surface with respect to the position of each angle without using only the illumination unit 3 in the above-described lymphatic system examination device 1, and is the relative intensity of the luminance value of the fluorescent image of the capillary tube when the fluorescence image of the capillary tube acquired under the condition is not corrected. As illustrated in FIG. 18, the result of "after correction" is closer to the result of "uniform irradiation" than the result of "before correction".

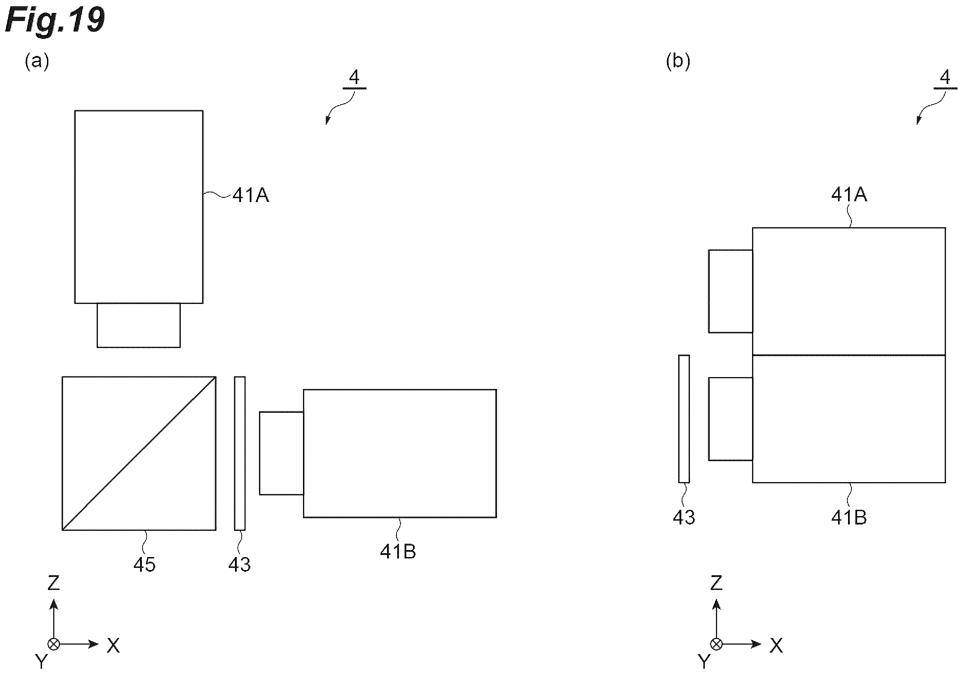

Note that the imaging unit 4 used in the correction processing described above may be configured as illustrated in (a) and (b) of FIG. 19.

The imaging unit 4 illustrated in (a) of FIG. 19 includes a first camera 41A, a second camera 41B, the filter 43, and a dichroic mirror 45. The first camera 41A has sensitivity to excitation light. The second camera 41B has sensitivity to fluorescence. The dichroic mirror 45 guides excitation light to the first camera 41A and guides fluorescence to the second camera 41B. The filter 43 selectively transmits the fluorescence between the dichroic mirror 45 and the second camera 41B. Note that the same camera having sensitivity to the excitation light and the fluorescence may be used as each of the first camera 41A and the second camera 41B. In addition, instead of the dichroic mirror 45, another optical element (for example, a prism and a half mirror) that guides excitation light to the first camera 41A and guides the fluorescence to the second camera 41B may be used. In addition, the light transmitting member 42 that transmits excitation light may be disposed between the dichroic mirror 45 and the first camera 41A.

The imaging unit 4 illustrated in (b) of FIG. 19 includes the first camera 41A, the second camera 41B, and the filter 43. The first camera 41A has sensitivity to excitation light. The second camera 41B has sensitivity to fluorescence. The first camera 41A and the second camera 41B are arranged in a state of facing the imaging region R side. The filter 43 selectively transmits the fluorescence at the preceding stage of the second camera 41B. Note that the same camera having sensitivity to the excitation light and the fluorescence may be used as each of the first camera 41A and the second camera 41B. In addition, the light transmitting member 42 that transmits excitation light may be disposed at the preceding stage of the first camera 41A.

According to the imaging unit 4 illustrated in (a) and (b) of FIG. 19, the excitation light image 201 of the subject S and the fluorescence image 203 of the subject S can be simultaneously acquired. Therefore, it is possible to correct the fluorescence image 203 based on the excitation light correction data 202 while the excitation light correction data 202 is generated based on the excitation light image 201. This is particularly effective in a case where a plurality of images arranged in time series is generated as the corrected fluorescence image of the subject S and the plurality of images is continuously displayed on the display unit 12.

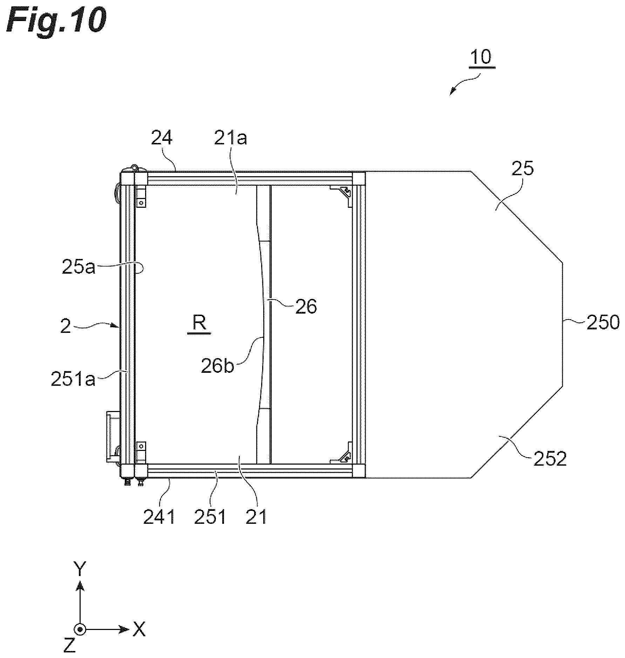

The present disclosure is not limited to the above embodiments. For example, as illustrated in FIG. 10, an external surface 26b of the restricting portion 26 on the side opposite to the imaging unit 4 (see FIG. 3) may be a curved surface curved along the lower abdomen of the subject S (that is, so as to be recessed toward the imaging unit 4 side). Also, the positioning portion 8 may be a recess or a protrusion provided on the placement surface 21a so as to match the foot rests of the subject S instead of the foot rests 81 and 82 or together with the foot rests 81 and 82. At least one of the illumination unit 3, the imaging unit 4, the distance sensor 5, the camera 6, and the restricting portion 26 may be attached to the frame 24 so as to enable at least one of position adjustment and angle adjustment.

### Reference Signs List

- 1: lymphatic system examination device
- 2: main body portion
- 3: illumination unit
- 4: imaging unit
- 5: distance sensor
- 6: camera
- 7: cover
- 8: positioning portion
- 11: control unit
- 12: display unit
- 14: first plate
- 15: scale
- 16: second plate
- 21: placement portion
- 22: support portion
- 25: wall portion
- 25a: opening
- 26: restricting portion
- 26a: external surface
- 31: first light emitting region
- 31a: first light emitting portion
- 32: second light emitting region
- 32a: second light emitting portion
- 81, 82: foot rest
- 250: housing
- 251: first wall portion
- 252: second wall portion
- L: lower body
- R: imaging region
- Ra: upper end portion
- S: subject

## Claims

1. A lymphatic system examination device comprising:
a main body portion including a placement portion on which a subject in which a fluorescent dye is injected into a lymphatic system stands, and configured to position a lower body of the subject in an imaging region on the placement portion;
an illumination unit configured to irradiate the lower body with excitation light; and
an imaging unit configured to detect fluorescence emitted from the lower body in response to irradiation of the excitation light,
wherein the illumination unit and the imaging unit are arranged on one side of the imaging region in a first horizontal direction,
the illumination unit includes a first light emitting region and a second light emitting region arranged in a state of being separated from each other in a second horizontal direction perpendicular to the first horizontal direction,
each of the first light emitting region and the second light emitting region extends in a vertical direction in a state of facing the imaging region, and
the imaging unit faces the imaging region across a region between the first light emitting region and the second light emitting region.

2. The lymphatic system examination device according to claim 1,
wherein the imaging unit is positioned in a center of the imaging region when viewed from the first horizontal direction.

3. The lymphatic system examination device according to claim 1 or 2,
wherein the illumination unit is positioned between the imaging region and the imaging unit in the first horizontal direction.

4. The lymphatic system examination device according to any one of claims 1 to 3,
wherein the first light emitting region includes a plurality of first light emitting portions,
the second light emitting region includes a plurality of second light emitting portions, and
the illumination unit has a function of adjusting a light emission intensity of each of the plurality of first light emitting portions and a light emission intensity of each of the plurality of second light emitting portions.

5. The lymphatic system examination device according to any one of claims 1 to 4,
wherein the first light emitting region and the second light emitting region are arranged so as to be away from each other in the second horizontal direction as approaching the imaging region in the first horizontal direction.
